# EUROPEAN PATENT APPLICATION

(11) **EP 3 034 622 A1**
(43) Date of publication of application: **22.06.2016**
(21) Application number: 14307115.7
(22) Date of filing: 19.12.2014
(51) Int. Cl.: C12Q 1/68

(54) **Genomic classifier that predicts response to multi-kinase inhibitor treatment Introduction**

(71) Applicant: Centre Léon Bérard, 69373 Lyon Cedex 08 (FR)
(72) Inventor: Blay, Jean-Yves, 69300 Caluire (FR); Jiang, Xiaojun, 69002 Lyon (FR); Tredan, Olivier, 69130 Ecully (FR); Ray Coquard, Isabelle, 69300 Caluire (FR)
(74) Representative: Colombet, Alain André

(57) **Abstract**

The method for predicting the anti-tumor response in a human or animal having a tumor to multiple kinase inhibitors, using any multiple kinase inhibitor, comprises selection of genes encoding for protein kinases targeted by the said tyrosine kinase inhibitor, for each one of these genes, providing at least one nucleic acid probe which hybridizes to said gene under stringent conditions, thus providing an array of nucleic acid probes, having a biological sample containing cancer cells from said human or animal, extracting DNA from the sample, fragmenting into DNA fragments, optionally labeling the DNA fragments, submitting the optionally labeled DNA fragments to hybridization with the array of nucleic acid probes, recovering and quantifying for all the genes the gains or losses in gene copy numbers, wherein gains and losses of gene copy numbers of each selected gene are used to determine whether the tumor is sensitive or not to said kinase inhibitor.

## Description

### Introduction

The present invention is related to a method for predicting the anti-tumor response in a human or animal to any multiple tyrosine kinase inhibitor (MTKI). The invention also relates to a method combining prediction and treatment.

Small molecule antiangiogenic tyrosine kinase inhibitors (TKI), such as regorafenib, sorafenib, sunitinib, pazopanib, axitinib, cabozantinib, represent a growing family of cancer treatment active in a variety of advanced cancers, from renal cell carcinoma, gastrointestinal stromal tumors (GIST), hepatocellular carcinoma (HCC), colorectal cancer (CRC), thyroid cancers 1,2,3,4,5,6,7,8,9. These TKI are broad-spectrum oral multi-kinase inhibitors, targeting multiple membrane-bound and intracellular kinases involved in normal cellular functions and contributing to pathologic processes 10, 11.

Compared with single-targeted agents, such as monoclonal antibodies (mAbs), these kinase inhibitors affect therefore multiple protein targets in cancer cells as well on surrounding cells of the tumor stroma. Predictive criteria for response to these multiple kinase inhibitors are not as well determined as for tumors harboring key driver alterations, such as BCR-ABL-positive in chronic myeloid leukemia (CML), KIT-mutant GIST, BRAF-mutant melanoma, ALK-positive non-small cell lung cancer and others 12,13,14,15,16,17. The median progression free survival (PFS) is often in the range of months. It is therefore shorter than that achieved with TKIs directed against strong driver molecular alterations such as chronic myeloid leukemia or GIST. Regorafenib for instance has been demonstrated in pretreated metastatic colorectal cancer (mCRC) to yield an improvement in PFS of 0.9 weeks and 4 months in imatinib and sunitinib refractory gastrointestinal stromal tumors (GIST) 9, 18, 19.

A number of approaches to identify biomarkers, such as measuring circulating cytokines related to angiogenesis or drug exposure have been reported with limited successes so far to predict responders and patients benefiting from these MTKI treatments 20, 21, 22,23,24,25. Recently, predictive signatures for response to regorafenib based on the plasma proteins were also proposed 26. No validated parameter was found capable yet to predict the response to multitarget tyrosine kinase inhibitors.

The identification of predictive marker for efficacy, or primary resistance, would be of obvious value in the approved indications of these treatments, but also to explore more efficiently the anecdotal antitumor activities of these agents on other tumors types. Biological parameters governing tumor response may be shared across tumor types. A general paradigm to identify predictive factors for response to these MTKIs would be of important clinical value.

It is well acknowledged that most tumors are associated with complex genetic alterations, with gains losses, and mutations of variety of genes, and that the acquisition of cancer cell phenotype relies on the acquisition of multiple oncogenic events and, with oncogene activation, gains, amplification and/or tumor suppressor gene losses 11. The sum of the complex losses and gains of genes and chromosomal segments observed in cancer cells have been recently shown to contribute to the acquisition of cancer cell phenotype and progression.

An objective of the invention is to provide for a method for predicting the anti-tumor response in a human or animal to any multiple kinase inhibitor.

Another objective of the invention is to provide for such a method that may be applied to any type of tumor sensitive to multiple kinase inhibitors.

Still another objective is to provide for a method that can be applied in clinic, and be combined with personalized therapeutic treatment.

The present invention is based on inventor's hypothesis that response to MTKI may be observed preferentially in cancer cells which acquired additional copies of the different genes encoding for the protein targets of these MTKI. An approach that analyses the sum of copy number gains and deletions of genes encoding for the proteins targeted by these MTKI to predict the response of MTKI therapy across different histological types has thus been developed.

The invention reports that the sum of gains and deletions of genes encoding for targets of multi-targeted (or multiple) tyrosine kinase inhibitors (MTKI) has a predictive value in patients treated with the MTKIs such as VEGFR tyrosine kinase inhibitors by an integrative approach incorporating high-resolution analyses of somatic DNA copy number aberrations (SCNAs) and possibly targeted mutation sequencing. The invention demonstrates that the treatment response variability is at least partially dependent on the SCNAs, especially those of genes encoding for tyrosine kinase proteins and receptors targeted by or sensitive to MTKIs. The feasibility has been first demonstrated on a cohort composed of mCRC patients treated with regorafenib and confirmed on a second validation cohort containing different tumors types (soft tissue sarcoma, thyroid carcinoma, hepatocellular carcinoma, renal cell carcinoma etc.) treated with other 6 MTKIs (sorafenib, sunitinib, pazopanib, axitinib, vandetanib and cabozantinib).

An object of the invention is thus a method for predicting the anti-tumor response to multiple kinase inhibitors in a human or animal having a tumor, using any multiple kinase inhibitor, comprising:
- selection of genes encoding for tyrosine kinases targeted by the said tyrosine kinase inhibitor,
- for each one of these genes, providing at least one nucleic acid probe which hybridizes to said gene under stringent conditions, thus providing an array of nucleic acid probes,
- having a biological sample containing cancer cells from said human or animal,
- extracting DNA from the sample, fragmenting into DNA fragments, optionally labeling the DNA fragments,
- submitting the optionally labeled DNA fragments to hybridization with the array of nucleic acid probes,
- recovering and quantifying for all the genes the gains or losses in gene copy numbers,
- wherein gains and losses of gene copy numbers of each selected gene are used to determine whether the tumor is sensitive or not to said kinase inhibitor.

By "tyrosine kinases", it is meant free proteins and bound proteins such as receptors, having a tyrosine kinase activity. Multi-targeted (or multiple) tyrosine kinase inhibitors (MTKI) means as usual that these inhibitors target several "tyrosine kinases". The known targets for a number of MKTIs is available to the skilled person, especially on DrugBank (www.drugbank.ca).

In order to get the most complete response, it is desirable to select all the genes (complete panel) encoding for tyrosine kinases sensitive (i.e. targeted by) to the assayed MKTI. In particular, the invention makes use of the complete available panel of genes encoding for tyrosine kinases targeted by the assayed MKTI. However, the person skilled in the art may appreciate that it is possible to get an acceptable result using an incomplete panel of genes in particular when the number of genes in the panel is high, such as in the case of regorafenib, sorafenib, sunitinib and pazopanib. Also, if additional genes encoding for tyrosine kinases targeted by the assayed MKTI would come to be discovered, these additional genes could and preferably would be included in the panel of genes.

The present invention may be applied to any known and future MKTI. For example, the invention may be applied to regorafenib, imatinib, sorafenib, sunitinib, pazopanib, axitinib, cabozantinib or vandetanib, but also MTKI targeting MET, ALK, Axl or other members of the human kinome.

**Table 1 gives the known genes encoding the protein targets of tested MKTIs.**

| Inhibitor name | **Marketed name** | **Reported Targets (complete panel)** |
|---|---|---|
| Sorafenib | Nexavar® | BRAF, RAF1, VEGFR3 (FLT4), VEGFR2(KDR), FLT3, PDGFRβ, KIT, RET, VEGFR1 (FLT1), FGFR1 |
| Sunitinib | Sutent® | VEGFR1 (FLT1), KIT, VEGFR2(KDR), VEGFR3(FLT4), FLT3, CSF1 R, PDGFRα, PDGFRβ |
| Pazopanib | Votrient® | VEGFR1 (FLT1), VEGFR2(KDR), VEGFR3(FLT4), PDGFRα, PDGFRβ, KIT, FGFR3, ITK/TSK, FGF1,SH2B3 |
| Regorafenib | Stivarga® | RET, VEGFR1 (FLT1), VEGFR2(KDR), VEGFR3(FLT4), KIT, PDGFRα, PDGFRβ, FGFR1, FGFR2, angiopoietin-1 receptor(TEK), DDR2, High affinity nerve growth factor receptor(NTRK1), EPHA2, RAF1, BRAF, MAPK11, FRK, ABL1 |
| Axitinib | Inlyta® | VEGFR1 (FLT1),VEGFR2(KDR), VEGFR3(FLT4),KIT, PDGFRβ |
| Vandetanib | Caprelsa® | VEGFR2(KDR),VEGF3(FLT4), VEGFA, EGFR, Protein-tyrosine kinase 6 (PTK6), angiopoietin-1 receptor(TEK) |
| Cabozantinib | Cometriq® | MET,VEGFR2(KDR), RET |

| | | |
|---|---|---|
| • BRAF: CSF1 R: macrophage colony-stimulating factor 1 receptor; DDR2: discoidin domain-containing receptor 2; EPHA2: Ephrin Type-A Receptor 2; FGFR: fibroblast growth factor receptor; FGF1: fibroblast growth factor 1;FRK: Tyrosine-protein kinase; ITK/TSK: IL-2 inducible T-cell kinase; MAPK11: mitogen-activated protein kinase 11; MET: Hepatocyte growth factor receptor; SH2B3: SH2B adaptor protein 3; VEGFA: vascular endothelial growth factor A. | | |

In a preferred embodiment, when the MKTI is regorafenib, sorafenib, sunitinib, pazopanib, axitinib, cabozantinib or vandetanib, the method makes use of the complete panel of genes sensitive to the MKTI as listed in Table 1.

The method preferably, but not exclusively, makes use of Comparative Genomic Hybridization (CGH).

The method comprises a phase of sample preparation. DNA is extracted from a tumor sample. This step is to prepare a population of nucleic acids of the genomic source to be analyzed.

Preferably, the CGH may employ at least a first and a second genomic populations. One of these populations may originate from the tumoral tissue or cells to be tested. The other may originate from healthy tissue or cells, and will serve as reference nucleic acid or DNA. In practicing the subject method, the first step may be to provide at least two different populations or collections of nucleic acids that are to be compared. The two or more populations of nucleic acids may or may not be labeled, depending on the particular detection protocol employed in a given assay. For example, in certain embodiments, binding events on the surface of a substrate may be detected by means other than by detection of a labeled nucleic acids, such as by change in conformation of a conformationally labeled immobilized oligonucleotide, detection of electrical signals caused by binding events on the substrate surface, etc. Typically, however, the populations of nucleic acids are labeled, where the populations may be labeled with the same label or different labels, depending on the actual assay protocol employed. For example, where each population is to be contacted with different but identical arrays, each nucleic acid population or collection may be labeled with the same label. Alternatively, where both populations are to be simultaneously contacted with a single array of immobilized oligonucleotide features, i.e., cohybridized to the same array of immobilized nucleic acid features or populations of nucleic acids that are to be compared are generally distinguishably or differentially labeled with respect to each other.

The two or more populations of nucleic acids are prepared from different genomic sources. As such, the first step is to prepare a collection of nucleic acids, e.g., labeled nucleic acids, from an initial genomic source for each genome that is to be compared.

The term genome refers to all nucleic acid sequences (coding and non-coding) and elements present in or originating from any animal or human. The term genome also applies to any naturally occurring or induced variation of these sequences that may be present in a mutant or disease variant of any cell type.

For example, the human genome consists of approximately 3.10⁹ base pairs of DNA organized into distinct chromosomes. The genome of a normal diploid somatic human cell consists of 22 pairs of autosomes (chromosomes 1 to 22) and either chromosomes X and Y (males) or a pair of chromosome Xs (female) for a total of 46 chromosomes. A genome of a cancer cell may contain variable numbers of each chromosome in addition to deletions, rearrangements and amplification of any subchromosomal region or DNA sequence.

The genomic source may be prepared using any convenient protocol. It may be prepared by first obtaining a starting composition of genomic DNA, e.g., a nuclear fraction of a cell lysate, where any convenient means for obtaining such a fraction may be employed and numerous protocols for doing so are well known in the art. The genomic source may be genomic DNA representing the entire genome from a particular organism, tissue or cell type. Alternatively, the genomic source may comprise a portion of the genome, e.g., one or more specific chromosomes or regions thereof, which optionally may have been PCR amplified with a pairs of specific primers.

A given initial genomic source may be prepared from a subject, which subject is suspected of being homozygous or heterozygous for a deletion or amplification of a genomic region. In certain embodiments, the average size of the constituent molecules that make up the initial genomic source typically have an average size of at least about 1 Mb, where a representative range of sizes is from about 50 to about 250 Mb or more, while in other embodiments, the sizes may not exceed 20 about 1 MB, such that they may be about 1 Mb or smaller, e.g., less than about 500 Kb, etc.

Where desired, the initial genomic source may be fragmented in the generation protocol, as desired, to produce a fragmented genomic source, where the molecules have a desired average size range, e.g., up to about 10 Kb, such as up to about 1 Kb, where fragmentation may be achieved using any convenient protocol, including but not limited to: mechanical protocols, e.g., sonication, shearing, chemical protocols, such as enzyme digestion.

The collection of nucleic acids that is prepared may then be labeled with a detectable label. A number of different nucleic acid labeling protocols are known in the art and may be employed to produce a population of labeled nucleic acids. The particular protocol may include the use of labeled primers, labeled nucleotides, modified nucleotides that can be conjugated with different dyes, one or more amplification steps, etc. The person skilled in the art may thus refer to US 8,232,055.

In one type of representative labeling protocol of interest, the initial genomic source, which most often is fragmented, may be employed in the preparation of labeled nucleic acids as a genomic template from which the labeled nucleic acids are enzymatically produced. Different types of template dependent labeled nucleic acid generation protocols are known in the art. The template may thus be employed in a non-amplifying primer extension nucleic acid generation protocol. The template may alternatively be employed in an amplifying primer extension protocol. In an embodiment, a non-enzymatic labeling method which is widely known is called the Universal Linkage System (ULS).

In generating labeled nucleic acids, the above-described genomic template and random primer population may be employed together in a primer extension reaction that produces the desired labeled nucleic acids. Primer extension reactions for generating labeled nucleic acids are well known to those of skill in the art, and any convenient protocol may be employed. The primer may be contacted with the template under conditions sufficient to extend the primer and produce a primer extension product, either in an amplifying or in a non-amplifying manner. As such, the above primers are contacted with the genomic template in the presence of a sufficient DNA polymerase under primer extension conditions sufficient to produce the desired primer extension molecules. DNA polymerases of interest include, but are not limited to, polymerases derived from E. coli, thermophilic bacteria, archaebacteria, phage, yeasts, Neurosporas, Drosophilas, primates and rodents. The DNA polymerase extends the primer according to the genomic template to which it is hybridized in the presence of additional reagents which may include, but are not limited to: dNTPs; monovalent and divalent cations, e.g. KCI, MgCl₂; sulfhydryl reagents, e.g. dithiothreitol; and buffering agents, e.g. Tris-Cl.

The labeling reagent may be a primer or a labeled nucleotide, which may be labeled with a directly or indirectly detectable label. A directly detectable label is one that can be directly detected without the use of additional reagents, while an indirectly detectable label is one that is detectable by employing one or more additional reagent, e.g., where the label is a member of a signal producing system made up of two or more components. The label may be a directly detectable label, such as a fluorescent label, where the labeling reagent employed in such embodiments is a fluorescently tagged nucleotide(s), e.g., dCTP. Fluorescent moieties which may be used to tag nucleotides for producing labeled nucleic acids include, but are not limited to: fluorescein, the cyanine dyes, such as Cy3, Cy5, Alexa 555, Bodipy 630/650, and the like. Other labels may also be employed as are known in the art.

In the next step, the collections or populations of labeled nucleic acids may be contacted to a plurality of different surface immobilized elements (i.e., features) under conditions such that nucleic acid hybridization to the surface immobilized elements can occur. The collections can be contacted to the surface immobilized elements either simultaneously or serially. The compositions may be contacted with the plurality of surface immobilized elements, e.g., the array of distinct oligonucleotides of different sequence, simultaneously. The method may thus comprise mixing the optionally labeled DNA fragments with optionally labelled non-tumoral reference DNA and subjecting the mixture to CGH.

Surface immobilized nucleic acids that make up the features of the arrays employed in such applications can be derived from virtually any source. Typically, the nucleic acids will be nucleic acid molecules having sequences derived from representative locations along a chromosome of interest, a chromosomal region of interest, an entire genome of interest, a cDNA library, and the like.

Preferably, the assay of nucleic acid probes is an array of oligonucleotides. The array comprises either oligonucleotides or other nucleic acid probes representative of the whole genome, or of previously identified regions from one or several chromosomal regions of interest, i.e. those regions that contain the genes encoding for the proteins targeted by the MKTI.

The oligonucleotides may be single stranded nucleotide multimers. The oligonucleotides may preferably be from about 10, 50 or 100 nucleotides up to about 200 nucleotides in length.

The oligonucleotides may preferably be bound to a surface of a solid support in a feature or spot. The support may have a variety of configurations, e.g., a sheet, bead or other structure. In certain embodiments, the collections of features of oligonucleotides are present on a surface of the same planar support, in the form of an array. The term "array" encompasses the term "microarray" and refers to an ordered array presented for binding to nucleic acids. The person skilled in the art may refer to US 8,232,055.

In a preferred embodiment, the method uses whole-genome oligonucleotide arrays, representative of the genome of the animal or human. In an embodiment, the method uses a Human whole-genome oligonucleotide arrays, preferably presented on microarrays, such as the one presented in the examples comprising about 180 000 oligonucleotide probes. One may use the commercial human genome expression microarrays, such as those available before Agilent Technologies.

The copy number of particular nucleic acid sequences in the two collections may then be compared by hybridizing the collections to the nucleic acid, preferably oligonucleotide, arrays, as described above. The hybridization signal intensity, and the ratio of intensities, may be determined.

Standard hybridization techniques (using high stringency hybridization and washing conditions) are used to assay a nucleic acid array. Suitable methods of CGH are referred to in US 8,232,055 already mentioned above, and the person skilled in the art may refer to this US patent, the disclosure of which is herein incorporated by reference.

Generally, nucleic acid hybridizations comprise the following major steps: (1) provision of array of surface immobilized nucleic acids or features; (2) hybridization of the mixture of nucleic acids to the features on the solid surface, typically under high stringency conditions; (3) post-hybridization washes to remove nucleic acid fragments not bound in the hybridization; and (4) detection of the hybridized nucleic acid fragments.

As indicated above, hybridization is carried out under suitable or selective stringent hybridization conditions, which may vary in stringency as desired. In certain embodiments, highly stringent hybridization conditions may be employed. The term "stringent assay conditions" as used herein refers to conditions that are compatible to produce binding pairs of nucleic acids, e.g., surface bound and solution phase nucleic acids, of sufficient complementarity to provide for the desired level of specificity in the assay while being less compatible to the formation of binding pairs between binding members of insufficient complementarity to provide for the desired specificity. Stringent assay conditions are the summation or combination (totality) of both hybridization and wash conditions.

A "stringent hybridization" and "stringent hybridization wash conditions" in the context of nucleic acid hybridization (e.g., as in array, Southern or Northern hybridizations) are sequence dependent, and are different under different experimental parameters. Stringent hybridization conditions that can be used to identify nucleic acids within the scope of the invention can include, e.g., hybridization in a buffer comprising 50% formamide, 5xSSC, and 1% SDS at 42°C, or hybridization in a buffer comprising 5xSSC and 1% SDS at 65°C, both with a wash of 0.2xSSC and 0.1% SDS at 65°C. Exemplary stringent hybridization conditions can also include a hybridization in a buffer of 40% formamide, 1 M NaCl, and 1% SDS at 37°C., and a wash in 1xSSC at 45°C. Alternatively, hybridization to filter-bound DNA in 0.5 M NaHPO4, 7% sodium dodecyl sulfate (SDS), 1 mM EDTA at 65°C, and washing in 0.1xSSC/0.1% SDS at 68°C can be employed. Yet additional stringent hybridization conditions include hybridization at 60°C or higher and 3xSSC (450 mM sodium chloride/45 mM sodium citrate) or incubation at 42°C in a solution containing 30% formamide, 1 M NaCl, 0.5% sodium sarcosine, 50 mM MES, pH 6.5. Those of ordinary skill will readily recognize that alternative but comparable hybridization and wash conditions can be utilized to provide conditions of similar stringency.

Following hybridization, the surface of immobilized nucleic acids may typically be washed to remove unbound nucleic acids. Washing may be performed using any convenient washing protocol, where the washing conditions are typically stringent. Wash conditions used to identify nucleic acids may include, e.g.: a salt concentration of about 0.02 molar at pH 7 and a temperature of at least about 50°C or about 55°C to about 60°C; or, a salt concentration of about 0.15 M NaCl at 72°C for about 15 minutes; or, a salt concentration of about 0.2xSSC at a temperature of at least about 50°C or about 55°C. to about 60°C for about 15 to about 20 minutes; or, the hybridization complex is washed twice with a solution with a salt concentration of about 2xSSC containing 0.1% SDS at room temperature for 15 minutes and then washed twice by 0.1xSSC containing 0.1% SDS at 68°C for 15 minutes; or, equivalent conditions. Stringent conditions for washing can also be, e.g., 0.2xSSC/0.1 % SDS at 42°C.

A specific example of stringent assay conditions is rotating hybridization at 65°C and 20 rpm during 24 hours in a salt based hybridization buffer with a total monovalent cation concentration of 1.5 M followed by washes of 0.5xSSC and 0.1xSSC at 37°C.

Following hybridization and washing, the hybridization of the labeled nucleic acids to the array may be detected using standard techniques so that the surface of immobilized features, e.g., array, is read. Reading of the resultant hybridized array may be accomplished by illuminating the array and reading the location and intensity of resulting fluorescence at each feature of the array to detect any binding complexes on the surface of the array. For example, a scanner may be used for this purpose which is similar to the AGILENT Surescan scanner available from Agilent Technologies, Palo Alto, CA. Other suitable devices and methods are available. However, arrays may be read by any other method or apparatus, with other reading methods including other optical techniques (for example, detecting chemiluminescent or electroluminescent labels) or electrical techniques. The method is sufficiently sensitive to detect a single copy number difference or change in the amount of a sequence of interest between two samples.

In an embodiment, slides are washed, dried and scanned on a suitable scanner, such as the Agilent Surescan scanner, according to the manufacturer's recommendations.

Scanned images may be processed and analyzed using suitable softwares. For example, scanned images may be processed using Agilent Feature Extraction software V11.0 and the analysis carried out using the Agilent Genomic Workbench software V7.0. The identification of aberrant copy number segments may be based on a suitable commercial algorithm, such as ADM-2 segmentation algorithm with default settings (Threshold of 15.0). A null Log2 ratio corresponds to a balanced tumor / normal DNA ratio. Low-level and high-level copy number gains / losses were defined as a log2 (ratio) > 0.25 and 1.5.

The method may thus comprise treatment of the readings in order to calculate target gene copy number gains and losses in the tumor cells or tissue. The method may typically comprise the following steps:
- the sum of the copy number gains of all selected genes encoding for protein kinases targeted by said kinase inhibitor is calculated, it is called herein tumor target charge (TTC);
- the sum of the copy number losses of all selected genes encoding for protein kinases targeted by said kinase inhibitor is calculated, it is called tumor target loss (TTL).

The method may then comprise the interpretation of the results in accordance with an algorithm.

According to a feature, a TTC ≥ 4 and TTC ≥ TTL is indicative that the tumor is sensitive to the kinase inhibitor.

According to a feature, a TTC ≥ 4 and TTC < TTL is indicative that the tumor is resistant to the kinase inhibitor.

According to a feature, a TTC such as 1 <TTC<4 combined to TTC > TTL is indicative that the tumor is sensitive to the kinase inhibitor.

According to a feature, a TTC such as 1 <TTC<4 combined to TTC ≤ TTL is indicative that the tumor is resistant to the kinase inhibitor.

According to a feature, a TTC ≤ 1 is indicative that the tumor is resistant to the kinase inhibitor.

These features may be combined to for a complete algorithm. Preferably, the following algorithm called SUMSCAN may be used:

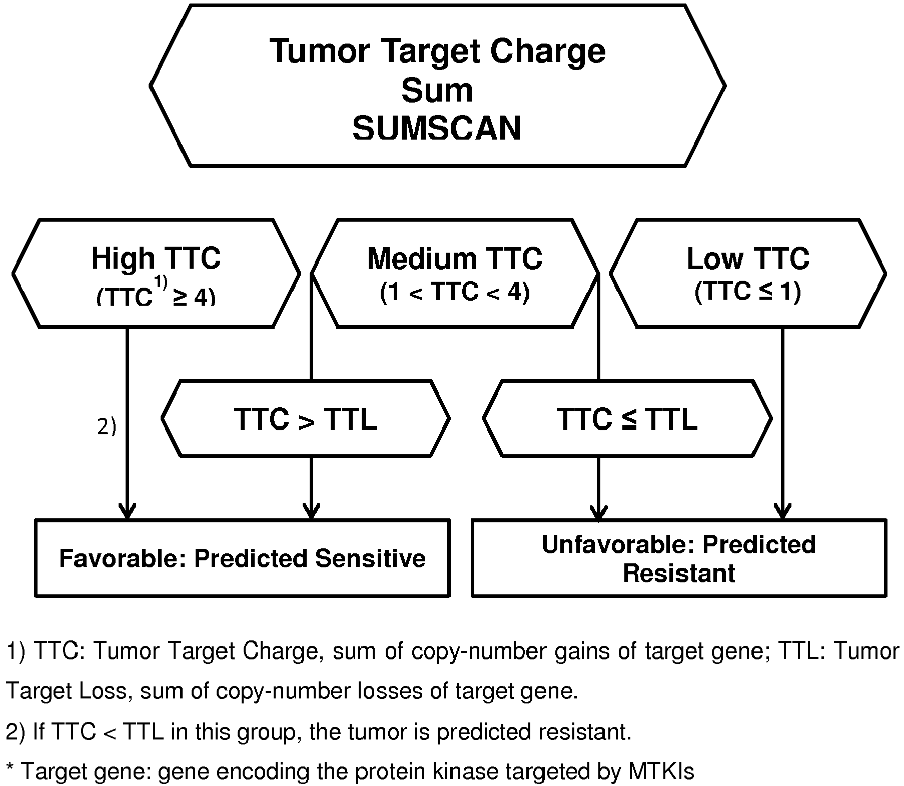

Another object of the invention is a multi-kinase inhibitor for use in treating a cancer in a human or animal subject that has been predicted sensitive to the multi-kinase inhibitor, using any suitable prediction method, in particular that has been predicted sensitive to the multi-kinase inhibitor by Comparative Genomic Hybridization (CGH), such as the one described herein.

Another object of the invention is a method of treating a cancer in a human or animal subject that has been predicted sensitive to the multi-kinase inhibitor, using any suitable prediction method, in particular that has been predicted sensitive to the multi-kinase inhibitor by Comparative Genomic Hybridization (CGH), such as the one described herein. In an embodiment, the subject is first submitted to a method of prediction as disclosed herein, and if the subject is predicted sensitive to the multi-kinase inhibitor, the method comprises the administration of an efficient amount of said multi-kinase inhibitor or to a therapeutic protocol comprising the administration of said multi-kinase inhibitor.

Also provided are kits for use in the subject invention, where such kits may comprise containers, each with one or more of the various reagents/compositions utilized in the method. A container comprises typically a collection of immobilized oligonucleotide features, e.g., one or more arrays of oligonucleotide features. Another container(s) may comprise labeling reagents for making two or more collections of distinguishably labeled nucleic acids. Another container may comprise a hybridization solution. Still another container may comprise a washing solution. Finally, the kits may further include instructions for using the kit components in the subject method.

The present invention will now be described in more detail using non-limiting embodiments referring to the appended figures.

Figure 1 is a graph presenting the sum of total gains and losses in regorafenib sensitive and resistant tumors (R = resistant, S = sensitive).

Figures 2 and 3 are graphs presenting respectively progression free survival (PFS, or progression free time) and overall survival (OS) curves of 25 patients treated with regorafenib.

Figures 4 and 5 are graphs presenting respectively gain and loss frequencies in regorafenib sensitive vs resistant tumors in the 25 tumors treated with regorafenib as 1^{st} line MTKI. The 18 genes targeted by regorafenib are concerned.

Figure 6 is a graph presenting the sum of total gains and losses in MKTI sensitive and resistant tumors (R = resistant, S = sensitive).

Figures 7-10 are graphs presenting progression free survival (PFS) and overall survival (OS) curves of patients treated with different MKTIs.

Figures 11-12 the number of accurate and inaccurate predictions in different tumor types classified by TTC and by SUMSCAN, respectively.

### Example 1:

### 1. Methods

### Study design and patients

Patients included in the profiLER program (Program to Establish the Genetic and Immunologic Profile of Patient's Tumor for All Types of Advanced Cancer, NCT01774409) treated with MTKI in advanced stage were included. The profiLER study enrolls patients with advanced solid tumors and aims to establish a genetic profile by CGH and targeted mutation sequencing. As of Nov 2014, 1163 patients have been included.

### Patients

58 patients were analyzed in this work

### Patients treated with regorafenib, n=25

Among the first 700 patients enrolled in the program from March 2013 to March 2014, 23 patients with metastatic colorectal cancer (mCRC) and 5 patients with advanced soft tissue sarcomas (STS) pretreated with chemotherapy received regorafenib from February 2011 to February 2014 **(Tables 2 and 3)** under the ATU, a compassionate use procedure of the French National Agency of Medicine and Health Products Safety. Three tumor samples did not fulfill DNA quality requirements for analysis. Twenty five tumor samples were therefore analyzed. This group was split into a discovery cohort of 13 CRC patients and the first validation cohort of 12 patients, with 7 mCRC and 5 STS patients. The patients received regorafenib at standard dose of 160mg or 120mg daily as first-line MTKI according the performance status. The regorafenib dosage was adjusted by the treating physician on the basis of presence / absence of adverse events.

### Patients treated with other MTKI, n=33

To generalize our hypothesis in other tumors types treated with other MTKIs, a second validation cohort of 33 profiLER patients treated with one of the 6 MTKIs (Sorafenib, Sunitinib, Pazopanib, Axitinib, Vandetanib and Cabozantinib) as 1^{st} line MTKI therapy. The predictive model was finally tested in a third set of 22 of these 33 patients who have received 2^{nd} or more MTKI.

| **TABLE 2** | **Discovery cohort** | **1^{st} Validation** | **2^{nd} Validation** |
|---|---|---|---|
| **Total** | 13 | 12 | 33 |
| **Age (median, range)** | 63.1 (40.7 - 75.8) | 56.0 (41.6 - 70.5) | 55.9 (24.6-76.1) |
| **Main tumor type n (%)** | | | |
| **CRC¹⁾** | 13 (100%) | 7 (58.3%) | 3 (9.1%) |
| **STS²⁾** | 0 (0%) | 5 (41.7%) | 5 (15.2%) |
| **RCC³⁾** | 0 (0%) | 0 (0%) | 12(36.3%) |
| **Thyroid⁴⁾** | 0 (0%) | 0 (0%) | 7 (21.2%) |
| **HCC⁵⁾** | 0 (0%) | 0 (0%) | 4 (12.1%) |
| **MTKIs concerned n (%)** | | | |
| **Regorafenib** | 13 (100%) | 12 (100%) | 0 (0%) |
| **Sorafenib** | 0 (0%) | 0 (0%) | 16 (48.4%) |
| **Sunitinib** | 0 (0%) | 0 (0%) | 12 (36.4%) |
| **Pazopanib** | 0 (0%) | 0 (0%) | 1 (3.0%) |
| **Axitinib** | 0 (0%) | 0 (0%) | 1 (3.0%) |
| **Vandetanib** | 0 (0%) | 0 (0%) | 2(6.1%) |
| **Cabozantinib** | 0 (0%) | 0 (0%) | 1 (3.0%) |
| **Baseline ECOG score n (%)** | | | |
| **0** | 2 (15.4%) | 5 (41.7%) | 13 (39.4%) |
| **1** | 4 (30.8%) | 3 (25%) | 14 (42.4%) |
| **2** | 5 (38.5%) | 2 (16.7%) | 2 (6.1%) |
| **NA ⁶⁾** | 2 (15.4%) | 2 (16.7%) | 4 (12.1%) |

| | | | |
|---|---|---|---|
| 1) CRC: Colorectal cancer 2)STS: Soft Tissue Sarcoma 3) RCC: Renal Cell Carcinoma 4) Thyroid: thyroid carcinoma 5) HCC: Hepatocellular Carcinoma 6) NA: not available | | | |

| **Table 3** | | | | | | |
|---|---|---|---|---|---|---|
| **N°** | **Gender** | **Age** | **Tumor Type** | **Time to metastasis (month)** | **MTKI received** | **Number of previous lines of chemotherapy** |
| **Discovery Cohort** | | | | | | |
| 1 BR | M | 64.5 | Colorectal | 14.1 | Regorafenib | 2 |
| 2 LJ | F | 61.5 | Colorectal | 7.6 | Regorafenib | 5 |
| 3 CB | F | 65.4 | Colorectal | 25.1 | Regorafenib | 10 |
| 4 DH | M | 72.2 | Colorectal | 12.0 | Regorafenib | 5 |
| 5 CP | M | 69.0 | Colorectal | 0 | Regorafenib | 4 |
| 6 BZ | F | 53.2 | Colorectal | 34.3 | Regorafenib | 4 |
| 7 JJ | F | 60.2 | Colorectal | 0 | Regorafenib | 2 |
| 8 BM | M | 69.7 | Colorectal | 0 | Regorafenib | 5 |
| 9 MB | M | 72.7 | Colorectal | 0 | Regorafenib | 5 |
| 10 RS | F | 40.7 | Colorectal | 0 | Regorafenib | 5 |
| 11 AM | M | 75.8 | Colorectal | 11.0 | Regorafenib | 3 |
| 12 MC | M | 65.4 | Colorectal | 0 | Regorafenib | 2 |
| 13 LH* | M | 51.2 | Colorectal | 1.9 | Regorafenib | 4 |

| **1^{st} Validation Cohort** | | | | | | |
|---|---|---|---|---|---|---|
| 1 PN | F | 55.1 | Colorectal | 0 | Regorafenib | >2 |
| 2 AS | F | 41.6 | Sarcoma | 23.1 | Regorafenib | 2 |
| 3 PA | M | 68.9 | Sarcoma | 4.4 | Regorafenib | 1 |
| 4 GN | F | 57.3 | Sarcoma | 0 | Regorafenib | 2 |
| 5 GE | F | 67.3 | Sarcoma | 5.3 | Regorafenib | 3 |
| 6 CJC | M | 53.4 | Colorectal | 0 | Regorafenib | 6 |
| 7 PE | F | 42.8 | Colorectal | NA | Regorafenib | 3 |
| 8 PN | F | 58.6 | Colorectal | 0 | Regorafenib | 2 |
| 9 NA | F | 70.5 | Colorectal | 28 | Regorafenib | 4 |
| 10 CF | F | 67.8 | Colorectal | 0 | Regorafenib | 3 |
| 11 BE | M | 44.1 | Sarcoma | 1.3 | Regorafenib | 2 |
| 12 RD | M | 44.4 | Colorectal | 0 | Regorafenib | 2 |

| **2^{nd} Validation Cohort** | | | | | | |
|---|---|---|---|---|---|---|
| 1 VOM | F | 53.9 | ACC⁴⁾ | 0 | Sunitinib | 2 |
| 2 NL* | F | 76.1 | Sarcoma | NA | Sorafenib | 2 |
| 3 DE | M | 45.3 | Colorectal | 0 | Sorafenib | 6 |
| 4 | F | 70.7 | Sarcoma | 0 | Sorafenib | 3 |
| 5 MC* | M | 47.1 | Thyroid | 4.1 | Sorafenib | 0 |
| 6 CC | F | 49.8 | RCC³⁾ | 86.4 | Sunitinib | 0 |
| 7 DP* | M | 53.4 | RCC | 20.4 | Sunitinib | 0 |
| 8 GAM | M | 69.1 | RCC | 23.8 | Sunitinib | 1 |
| 9 PJ* | M | 54.4 | Thyroid | 44.2 | Vandetanib | 0 |
| 10 TM | F | 65.6 | HCC | 5.0 | Sorafenib | 0 |
| | | | | | | |
| 11 BR* | M | 59.0 | Thyroid | 24.7 | Cabozantinib | 0 |
| 12 | M | 59.8 | RCC | 2 | Axitinib | 0 |
| 13 GS | M | 64.9 | CHC | 19.9 | Sorafenib | 0 |
| 14 PF | F | 62.9 | HCC¹⁾ | 6.5 | Sorafenib | 0 |
| 15 CC | F | 54 | Head&Neck | 0 | Pazopanib | 3 |
| 16 JD* | M | 55.1 | Sarcoma | 21.2 | Sorafenib | 3 |
| 17 DG* | M | 68.2 | Thyroid²⁾ | 0 | Sorafenib | 0 |
| 18 AJ | F | 50.4 | HCC | 4.5 | Sorafenib | 1 |
| 19 RJ* | M | 65.5 | RCC | 2.3 | Sunitinib | 0 |
| 20 GF | F | 43.5 | Colorectal | 0 | Sorafenib | 3 |
| 21 AL | F | 54.3 | RCC | 0 | Sunitinib | 0 |
| 22 AR* | M | 60.0 | RCC | 0 | Sunitinib | 0 |
| 23 NM | M | 49.9 | RCC | 1.6 | Sunitinib | 0 |
| 24 GR* | M | 51.7 | Thyroid | 15.7 | Vandetanib | 0 |
| 25 DC | M | 49.2 | Colorectal | 0 | Sorafenib | 3 |
| 26 | M | 56.4 | Thyroid | 36.9 | Sorafenib | 0 |
| 27 AJ* | M | 32.4 | Sarcoma | 5.7 | Sorafenib | 3 |
| 28 MM | F | 55.4 | Sarcoma | 135.1 | Sorafenib | 1 |
| 29 TG | M | 61.1 | Thyroid | 0 | Sorafenib | 0 |
| 30 FA* | M | 24.6 | RCC | 11.7 | Sunitinib | 0 |
| 31 MP* | M | 75.4 | RCC | 21.4 | Sunitinib | 0 |
| 32 TA* | F | 58.0 | RCC | 0 | Sunitinib | 0 |
| 33 MB* | M | 49.9 | RCC | 0 | Sunitinib | 0 |

| | | | | | | |
|---|---|---|---|---|---|---|
| 1). HCC: Hepatocellular Carcinoma 2). Thyroid: Differentiated thyroid carcinoma 3). RCC: Renal Cell Carcinoma 4). ACC: Adrenal Cortical CarcinomaPatients with * were treated by 2 or more MTKIs. | | | | | | |

### Response and progression free survival

All patients had progressive disease before initiation of MTKI. Patients included in this analysis had thoraco-abdomino-pelvic CT examinations performed at the center, 4+/-2 weeks before and 8+/-2 weeks after initiation of regorafenib or other MTKI treatment. Baseline demographic and clinical data were collected, with the site and dates of metastases, previous systemic therapies, MTKI treatment, treatment duration, date and results of follow-up imaging, responses and progression free survival (PFS) determined by the radiologist and physician at each follow-up visit with Response Evaluation Criteria in Solid Tumors (RECIST, version 1.1)27.

Best response during the treatment and the PFS (defined as time from the initiation of treatment to first radiological or clinical progression or death) were collected. Patients with complete response, partial response and stable disease lasting at least 2 months were defined as "MTKI sensitive"; those with progressive disease as best response at 2 months were classified as MTKI resistant.

### Array-Based Comparative Genomic Hybridization (CGH)

### Sample selection and DNA extraction

All the tumor samples (formalin fixed paraffin embedded- FFPE) were stored in the center before treatment by MTKIs. Tumor samples were collected from the primary tumor (n = 40, 69.0%) or from metastasis (n = 18, 31.0%), mainly from formalin fixed archival tissues. All samples were collected before the MTKI treatments.

### Array CGH

Fragmentation and labeling were done according to the manufacturer's recommendations for the CGH array (Agilent Technologies, Santa Clara, CA). In brief, 1.5 µg of tumor DNA and 1.5 µg of reference DNA (Promega #G1471 or #G1521, WI, USA) were heat denatured and fragmented during 10 min at 95°C. Then, tumor DNA was chemically labeled with Kreatech's Universal Linkage System (ULSTM) Cy5-dye, whereas reference DNA was labeled with Cy3-dye (Agilent #5190-0450). Labeled samples were then purified using KREApure columns (Agilent #5190-0418). Co-hybridization was performed on 4*180K Agilent SurePrint G3 Human whole-genome oligonucleotide arrays (Agilent #G4449A), containing 180 000 oligonucleotide probes. Slides were washed, dried and scanned on the Agilent Surescan scanner according to the manufacturer's recommendations. Scanned images were processed using Agilent Feature Extraction software V11.0 and the analysis was carried out using the Agilent Genomic Workbench software V7.0. The identification of aberrant copy number segments was based on ADM-2 segmentation algorithm with default settings (Threshold of 15.0). A null Log2 ratio corresponds to a balanced tumor / normal DNA ratio. Low-level and high-level copy number gains / losses were defined as a log2 (ratio) > 0.25 and 1.5.

### Somatic mutation detection with NGS (Ion Personal Genome Machine)

Ten nanograms of DNA were used for the Ion Torrent library preparation of a panel covering 59 key cancer genes (Table S2) following the manufacturer's protocol for the Ion AmpliSeq Library Kit 2.0 (Life Technologies). The size distribution of the DNA amplicons was analyzed on the 2200 TapeStation (Agilent) using the High sensitivity kit (Agilent). Template preparation, emulsion PCR, and Ion Sphere Particle (ISP) enrichment was performed using the One Touch 2 kit (Life Technologies) according to manufacturer's instructions. The ISPs were loaded onto a 318 chip (Life Technologies) and sequenced using an Ion PGM 200 v2 sequencing kit (Life Technologies) on the Ion Torrent PGM for 500 cycles.

After a successful sequencing reaction, the raw signal data were analyzed using NextGENe Software Suite v3.4.2 (Soft genetics). The pipeline includes quality score assignment, alignment to human genome 19 reference, mapping quality QC, coverage analysis and variant calling. After completion of the primary data analysis, lists of detected sequence variants (SNVs and INDELs) were compiled in a VCF (Variant Call File) format. For downstream analysis, variants with minimum coverage of 100 reads containing at least 10 of the mutant reads were selected. Variant calls were further analyzed using variant filtering and annotation using COSMIC v.64 and dbSNP build 135.

| | | | | | | | |
|---|---|---|---|---|---|---|---|
| ABL1 | CSF1 | IGF1 R | MET | PIK3CA | ROS1 | SRC | VEGFR1 |
| AKT1 | CSF1R | JAK2 | MPL | PIK3R1 | RYK | STK11 | VEGFR2 |
| AKT2 | DDB2 | JAK3 | MST1 R | PTCH | SDHAF2 | TEK | VEGFR3 |
| ALK | DDR1 | KIT | mTOR | PTEN | SDHB | TIE1 | VHL |
| APC | DDR2 | KRAS | MUSK | RB1 | SDHC | TP53 | |
| AXL | EGFR | NRAS | PDGFA | RET | SDHD | TSC1 | |
| BRAF | ERBB2 | HRAS | PDGFRA | ROR1 | SMARCB1 | TSC2 | |
| CRAF | FLT3 | MERTK | PDGFRB | ROR2 | SMO | TYRO3 | |

### Statistical analysis

The association between SCNAs and categorical variables was tested using the Mann-Whitney U-test. Association between categorical variables was assessed using Chi-square test. All p-values were two-sided. Survival curves were plotted using the Kaplan Meier method and compared using a log rank test. Statistical analysis was conducted using the SPSS 19.1 Package (SPSS, IBM France). Table 4: List of analysed mutations status by NGC (Ion torrent).

### 2. Results

### Patient characteristics

In total, all evaluable 58 patients included in the ProfilER study, who had received at least one of the 7 MTKIs listed in **Table 1** in the first line setting were included in this analysis. Patients' characteristics are detailed (**Tables 2 and 3**).

### Regorafenib treated patients: discovery cohort

25 patients treated with regorafenib as first-line MTKI. The discovery cohort consisted of 13 patients with metastatic colorectal cancers (mCRC) who had received the regorafenib as first-line MTKI treatment, after having progressed under irinotecan, oxaliplatin containing regimens. The median duration of regorafenib treatment was 3 months (range, 0.5 - 25). Six patients who had achieved a stable disease (SD) or objective response (partial response, PR or complete response, CR) at 8 weeks were considered as regorafenib sensitive and 7 other patients with progressive disease ≤ 8 weeks were qualified as regorafenib resistant.

### Establishment of the target copy number change pattern

The copy number changes of the panel of 18 genes encoding for kinases whose enzymatic activity is blocked by regorafenib was investigated : RET, VEGFR1 (FLT1), VEGFR2(KDR), VEGFR3(FLT4), KIT, PDGFRα, PDGFRβ, FGFR1, FGFR2, angiopoietin-1 receptor(TEK), DDR2, High affinity nerve growth factor receptor(NTRK1), EPHA2, RAF1, BRAF, MAPK11, FRK, ABL128. Analyzing the SCNA of 18 target genes in these 13 tumors using CGH array (Agilent), we explored a possible correlation between the clinical outcome and SCNAs of these target genes. The sum of gains on target genes were termed as tumor target charge (TTC), while the sum of deletions of target genes were termed as tumor target loss (TTL).

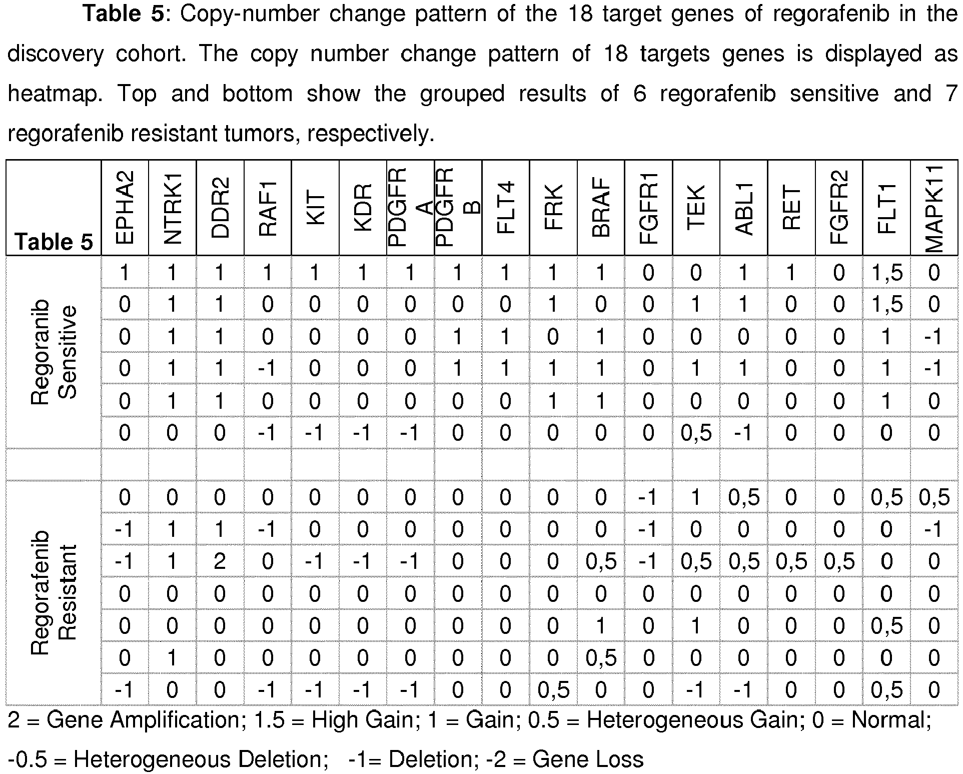

An enrichment in gains on genes encoding for regorafenib target was observed in sensitive patients with a total of 41 gains across 6 samples (mean: 6.8; range 1-14) versus 20 gains across 7 samples (mean: 2.1; range 0-7) in the regorafenib resistant group. The regorafenib sensitive tumors had a total of 8 deletions (mean: 1.3; range 0-5), while the resistant tumors had a total of 17 deletions (mean: 2.4; range 0-7). The differences between TTC and negative TTC were significantly higher in the sensitive group (P =0.038; Mann Whitney). In addition, five of six sensitive tumors had a TTC ≥ 4, vs 2 of 7 resistant tumors (P =0.048). The details of SCNAs of all patients are listed in **Table 6**.

| **Table 6 Patients characteristics, target gene copy number change and sensitivity predicted** | | | | | | |
|---|---|---|---|---|---|---|
| **Patients** | **Tumor** | **MTKIs** | **Best Response** | **Gains on targeted kinases** | **Losses on targeted kinases** | **Sensitivity Predicted** |
| **Discovery Cohort** | | | | | | |
| 1 BR | CRC¹⁾ | Regorafenib | SD | 14 | 0 | / |
| 2 LJ | CRC | Regorafenib | PR | 6 | 0 | / |
| 3 CB | CRC | Regorafenib | SD | 6 | 1 | / |
| 4 DH | CRC | Regorafenib | SD | 9 | 2 | / |
| 5 CP | CRC | Regorafenib | SD | 5 | 0 | / |
| 6 BZ | CRC | Regorafenib | SD | 0 | 0 | / |
| 7 AM | CRC | Regorafenib | PD | 2 | 0 | / |
| 8 BM | CRC | Regorafenib | PD | 2 | 4 | / |
| 9 MB | CRC | Regorafenib | PD | 2 | 5 | / |
| 10 MC | CRC | Regorafenib | PD | 1 | 0 | / |
| 11 JJ | CRC | Regorafenib | PD | 0 | 0 | / |
| 12 RS | CRC | Regorafenib | PD | 0 | 0 | / |
| 13 LH | CRC | Regorafenib | PD | 0 | 0 | / |

| **Validation Cohort I** | | | | | | |
|---|---|---|---|---|---|---|
| 1 GN | STS²⁾ | Regorafenib | SD | 14 | 0 | Sensitive |
| 2 PA | STS | Regorafenib | SD | 12 | 0 | Sensitive |
| 3 GE | STS | Regorafenib | SD | 7 | 1 | Sensitive |
| 4 AS | STS | Regorafenib | PR | 6 | 5 | Sensitive |
| 5 PN | CRC | Regorafenib | PR | 2 | 0 | Sensitive |
| 6 CF | CRC | Regorafenib | PD | 5 | 6 | Resistant |
| 7 PE | CRC | Regorafenib | PD | 5 | 6 | Resistant |
| 8 PN | CRC | Regorafenib | PD | 2 | 0 | Sensitive |
| 9 BE | STS | Regorafenib | PD | 2 | 2 | Resistant |
| 10 CJC | CRC | Regorafenib | PD | 1 | 4 | Resistant |
| 11 RD | CRC | Regorafenib | PD | 1 | 0 | Resistant |
| 12 NA | CRC | Regorafenib | PD | 0 | 0 | Resistant |
| 13 RD | CRC | Regorafenib | PD | 1 | 0 | Resistant |

| **Validation Cohort II** | | | | | | |
|---|---|---|---|---|---|---|
| 1 VOM | ACC³⁾ | Sunitinib | SD | 9 | 0 | Sensitive |
| 2 NL | STS | Sorafenib | SD | 4 | 2 | Sensitive |
| 3 DE | CRC | Sorafenib | SD | 3 | 0 | Sensitive |
| 4 DBO | STS | Sorafenib | SD | 3 | 1 | Sensitive |
| 5 MC | Thyroid ⁵⁾ | Sorafenib | SD | 3 | 1 | Sensitive |
| 6 CC | RCC⁴⁾ | Sunitinib | PR | 3 | 0 | Sensitive |
| 7 DP | RCC | Sunitinib | SD | 3 | 2 | Sensitive |
| 8 GAM | RCC | Sunitinib | SD | 3 | 0 | Sensitive |
| 9 PJ | Thyroid | Vandetanib | SD | 3 | 0 | Sensitive |
| 10 TM | HCC | Sorafenib | SD | 2 | 0 | Sensitive |
| 11 BR | Thyroid | Cabozantinib | PR | 2 | 0 | Sensitive |
| 12 BJM | RCC | Axitinib | SD | 2 | 1 | Sensitive |
| 13 GS | HCC | Sorafenib | SD | 2 | 0 | Sensitive |
| 14 PF | HCC⁶⁾ | Sorafenib | SD | 1 | 1 | Resistant |
| 15 CC | Head&neck | Pazopanib | SD | 1 | 0 | Resistant |
| 16 JD | STS | Sorafenib | SD | 0 | 2 | Resistant |
| 17 DG | Thyroid | Sorafenib | PR | 0 | 0 | Resistant |
| 18 AJ | HCC | Sorafenib | PR | 0 | 4 | Resistant |
| 19 RJ | RCC | Sunitinib | CR | 0 | 0 | Resistant |
| 20 GF | CRC | Sorafenib | SD | 0 | 2 | Resistant |
| 21 AL | RCC | Sunitinib | PD | 3 | 0 | Resistant |
| 22 AR | RCC | Sunitinib | PD | 3 | 0 | Resistant |
| 23 NM | RCC | Sunitinib | PD | 3 | 6 | Resistant |
| 24 GR | Thyroid | Vandetanib | PD | 1 | 2 | Resistant |
| 25 DC | CRC | Sorafenib | PD | 0 | 0 | Resistant |
| 26 VJL | Thyroid | Sorafenib | PD | 0 | 0 | Resistant |
| 27 AJ | STS | Sorafenib | PD | 0 | 3 | Resistant |
| 28 MM | STS | Sorafenib | PD | 0 | 3 | Resistant |
| 29 TG | Thyroid | Sorafenib | PD | 0 | 2 | Resistant |
| 30 FA | RCC | Sunitinib | PD | 0 | 3 | Resistant |
| 31 MP | RCC | Sunitinib | PD | 0 | 0 | Resistant |
| 32 TA | RCC | Sunitinib | PD | 0 | 0 | Resistant |
| 33 MB | RCC | Sunitinib | PD | 0 | 0 | Resistant |

| **Validation cohort III** | | | | | | |
|---|---|---|---|---|---|---|
| 1 PJ | Thyroid | Sunitinib | PR | 6 | 0 | Sensitive |
| 2 PJ | Thyroid | Sorafenib | SD | 6 | 0 | Sensitive |
| 3 DBO | STS | Regorafenib | SD | 5 | 1 | Sensitive |
| 4 NL | STS | Pazopanib | SD | 5 | 1 | Sensitive |
| 5 LP | GIST | Pazopanib | SD | 4 | 1 | Sensitive |
| 6 PF | HCC | Regorafenib | SD | 2 | 1 | Sensitive |
| 7 BR | Thyroid | Vandetanib | PR | 2 | 0 | Sensitive |
| 8 MC | Thyroid | Pazopanib | SD | 2 | 0 | Sensitive |
| 9 LP | GIST | Regorafenib | SD | 2 | 4 | Resistant |
| 10 DG | Thyroid | Pazopanib | SD | 0 | 0 | Resistant |
| 11 DP | RCC | Axitinib | SD | 0 | 0 | Sensitive |
| 12 LP | GIST | Sunitinib | PD | 3 | 2 | Sensitive |
| 13 TH | CRC | Regorafenib | PD | 1 | 0 | Resistant |
| 14 CJC | CRC | Sorafenib | PD | 1 | 1 | Resistant |
| 15 AJ | STS | Pazopanib | PD | 1 | 1 | Resistant |
| 16 LH | CRC | Sorafenib | PD | 0 | 0 | Resistant |
| 17 AR | RCC | Sorafenib | PD | 0 | 0 | Resistant |
| 18 JD | STS | Pazopanib | PD | 0 | 1 | Resistant |
| 19 VJL | Thyroid | Pazopanib | PD | 0 | 0 | Resistant |
| 20 GR | Thyroid | Sunitinib | PD | 0 | 8 | Resistant |
| 21 FA | RCC | Axitinib | PD | 0 | 2 | Resistant |
| 22 AR | RCC | Axitinib | PD | 0 | 0 | Resistant |
| 23 TA | RCC | Axitinib | PD | 0 | 0 | Resistant |
| 24 RJ | RCC | Axitinib | PD | 0 | 0 | Resistant |
| 25 MB | RCC | Axitinib | PD | 0 | 0 | Resistant |

### The regorafenib validation series

This difference of TTC and TTL was assessed in the 1 st validation cohort composed of the 12 patients treated with regorafenib as first-line MTKI. In this analysis, as well , gains of gene encoding for targets of regorafenib was observed in the sensitive tumors, as compared to resistant tumors (**Table 7**). The differences between TTC and TTL was significantly higher in sensitive tumors (Mann Whitney, p = 0.014). Additionally, four of five sensitive tumors had TTC ≥ 4, versus two of seven resistant tumors (P = 0.07; chi-square test) (**Table 7**). Even though several of these 18 individual genes gains predicted well for regorafenib sensitivity, the numerical combination of target gene gains and deletions was actually the most efficient predictor of sensitivity to regorafenib.

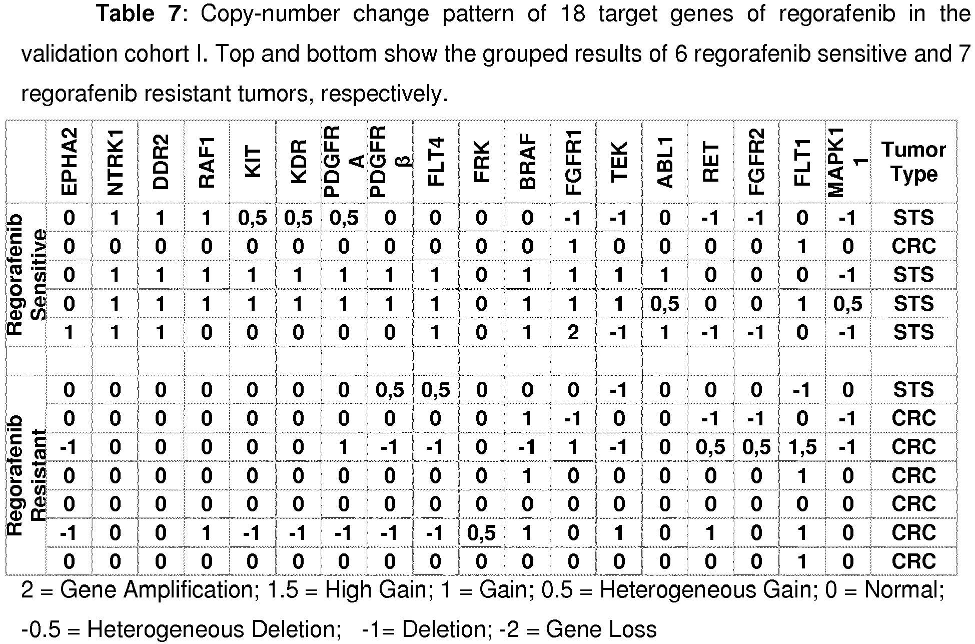

All gain events are considered as 1 TTC and all lost events are considered as 1 TTL.

### Pool of the regorafenib series

The pooled integral analysis of the 2 regorafenib sets (**Table 8**) revealed that the TTL tends to outnumber TTC in resistant tumors. As expected, the difference between TTC and TTL was significantly higher in the sensitive tumors (P = 0.003; Mann Whitney). Of note, all 8 patients with a difference no less than five achieved clinical benefit, vs 3 of the 17 remaining patients (p=0.0001). Combining the two parameters enabled to delineate an algorithm, termed as SUMSCAN (see above). Using this algorithm, ten of the eleven sensitive tumors would have been identified as responders, vs five of the fourteen remaining patients (P = 0.005; chi-square test), resulting in a sensitivity of 90.9% and a specificity of 66.7%. A prediction accuracy of 76% (19 of 25) was achieved.

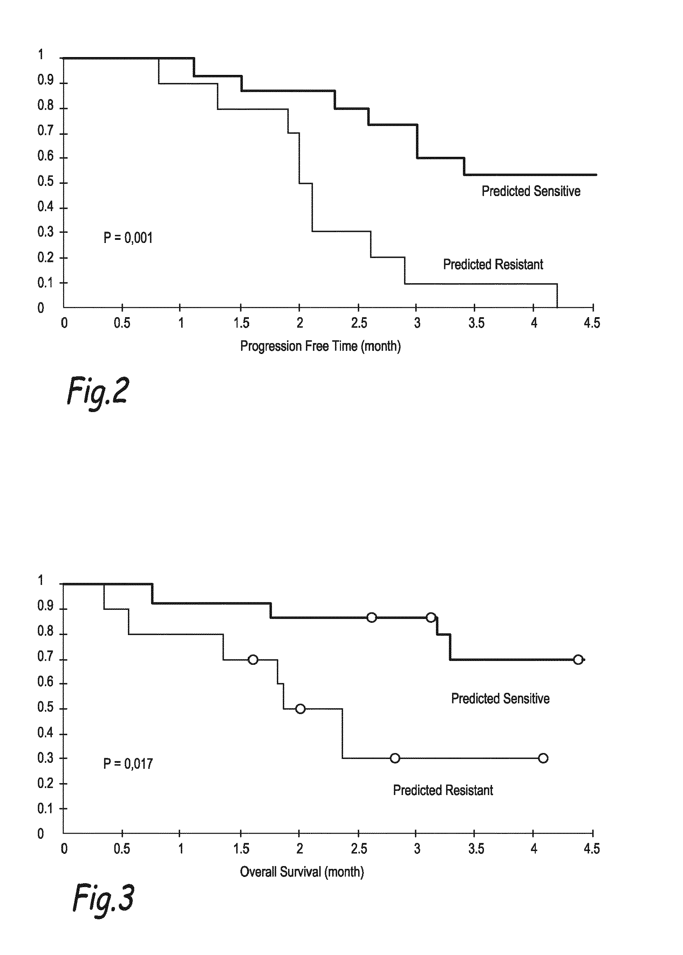

Furthermore, the prognostic significance of SUMSCAN was evaluated using univariate Kaplan-Meier survival analysis. The progression free survival and overall survival of patients treated with regorafenib in 2 cohorts are significantly better in patients with a favorable SUMSCAN profile (defined as TTC ≥ 4; P_{PFS} = 0.001, P_{OS} = 0.017, respectively; Log-rank test, **Fig. 2 and 3**).

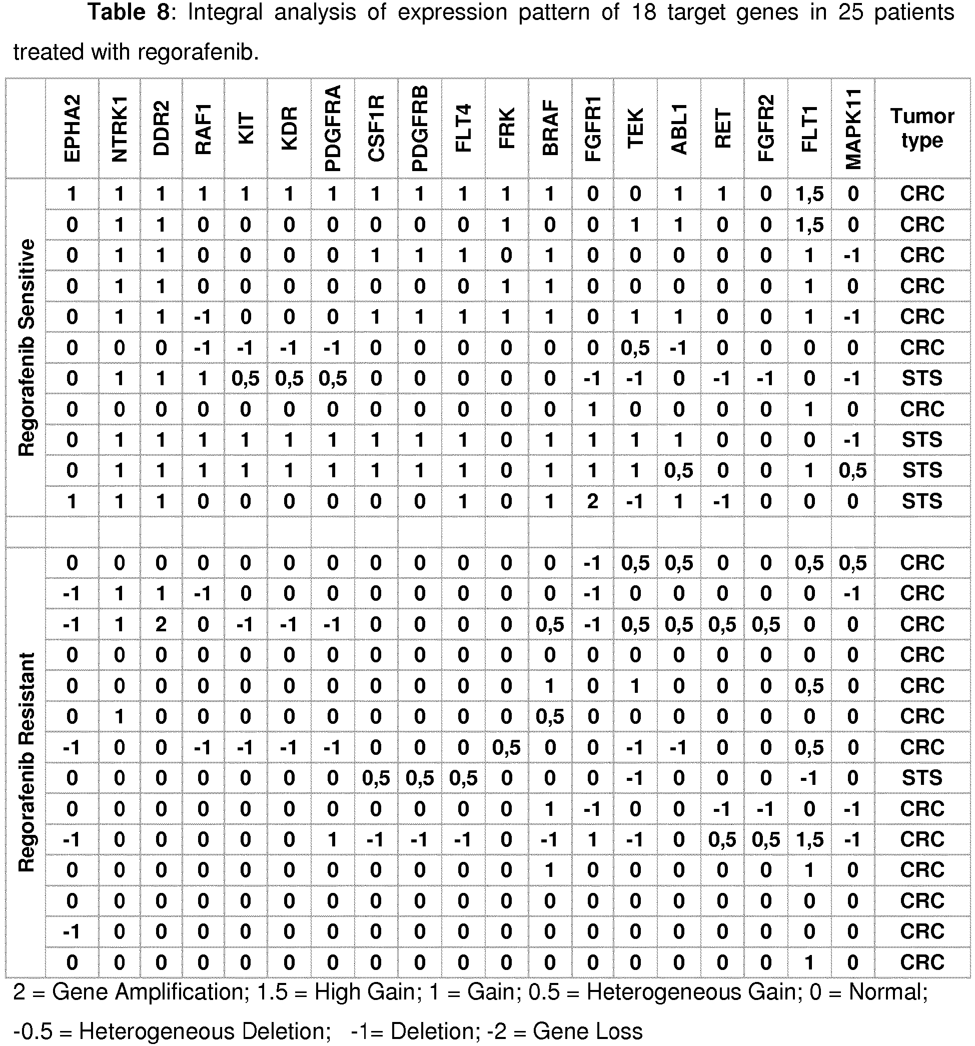

All gain events are considered as 1 TTC and all lost events are considered as 1 TTL.

### Gains and deletions of specific target genes

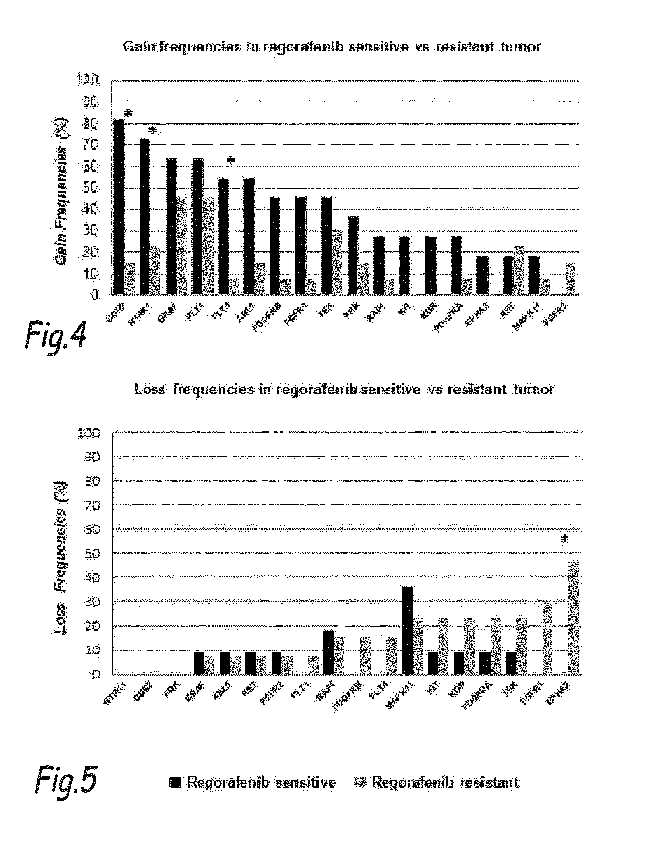

A significant difference in the gain frequencies between regorafenib sensitive tumor and regorafenib resistant tumors was observed for genes DDR2, NTRK1 (High affinity nerve growth factor receptor) and FLT4 (**Fig. 4**). Gains on DDR2, NTRK1 and FLT4 were observed in 81.8% (9/11), 72.7% (8/11) and 54.5% (6/11) of 11 sensitive tumors but only in 14.3% (2/14), 21.4% (3/14) and 7.1% (1/14) of the resistant tumors (P < 0.05 each, Fisher exact test) (**Fig. 4**). Additionally, specific deletion on EPHA2 was observed in 42.9% (6/14) of the resistant tumor but in none of the sensitive tumors (P = 0.013; chi-square test) (**Fig. 5**). No statistically significant association was observed for other 12 genes analyzed.

### Overall gains in the genome of sensitive vs resistant patients

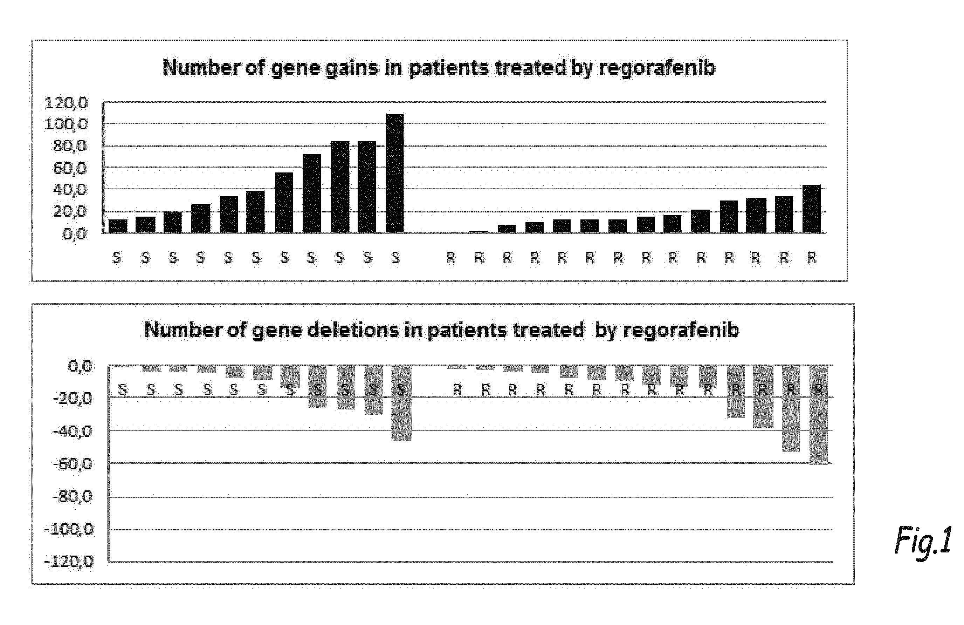

As a control, we compared whole genome gains and deletions in the same experiment (**Fig. 1**). A total of 545 gains across 187 genes were observed in the sensitive group (mean: 49.5; range 12-109). In the resistant group (n=14), a total of 246 gains across 187 genes was observed (mean: 16.7; range 0-44) (P =0.006; Mann-Whitney) showing that an overall gain profile is observed in responsive patients. As for the gene losses, a total of 175 losses in the sensitive group (mean = 15.9, range 1-46) versus 265 losses (mean = 18.9, range 2-61) in the resistant group were observed (P = 0.722; chi-square). The sum of gains minus losses of copies of the genes encoding for these genes which are not encoding for targets of regorafenib were not significantly higher in the group of patients with sensitive tumors (P =0.17, Mann Whitney).

### Added predictive ability of sequence mutations of the same samples using NGS

To gain more insight in the discrepancies between the SUMSCAN model and efficacy of the treatments, we then investigated the correlation between mutations in the genes of NGS panel (Gene list **Table 4**) and response to regorafenib. Target mutation sequencing was feasible for 100% (25 of 25) of the samples. PIK3CA mutation (42.9% in resistant tumors vs none of the sensitive tumors, P = 0.0196; Fisher exact test) was found associated with regorafenib resistance, but not TP53 (72.7% in sensitive tumor vs 64.3% in resistant tumors) nor KRAS (27.3% in sensitive tumor vs 57.1 % in resistant tumors). Two of six PIK3CA mutant tumors were predicted sensitive by SUMSCAN. Six PIK3CA hotspot mutations were located in exon 9 (E542K*2, E545K*2, Q546P) and one in exon 20 (H1047R), all with relatively low tumor target charge (TTC ≤ 2) and 5 of 6 predicted to be resistant by SUMSCAN. This association was observed only in mCRC, not in STS.

### Second validation series: Assessment of the model in tumors treated by other MTKIs

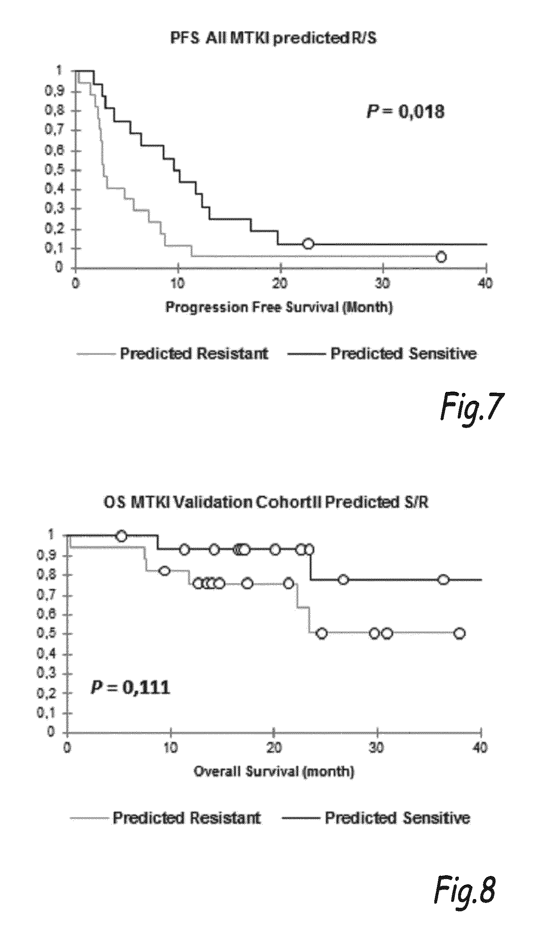

The prediction power of SUMSCAN was further assessed in 33 patients treated with other 6 MTKIs as 1^{st} line MTKI. Twenty-two patients have subsequently received 2 or more MTKIs (see **Table 6**). The definition of target genes varied between each MTKI according to DrugBank (http://www.drugbank.ca). The SUMSCAN was applied to this group of patients treated with different MTKI for a variety of neoplastic diseases (**Tables 6 and 8**). Again, the difference between TTC and TTL was significantly different between sensitive tumors (n = 20) and resistant tumors (n = 13) (P =0.008; Mann Whitney). Fourteen of twenty responsive patients were predicted sensitive by SUMSCAN, vs two of the thirteen resistant patients (P = 0.002; chi-square) resulting in a sensitivity of 70% and a predictive accuracy of 75.8%. The positive predictive value (PPV) and negative predictive value (NPV) were 87.5% and 64.7% respectively (P = 0.002, chi-square). Patients with a favorable SUMSCAN profile had a significantly better PFS, with a median PFS of 9.9 months vs only 2.8 months for patients with unfavorable SUMSCAN profile and a trend for a longer survival (**Fig. 7 and 8**)

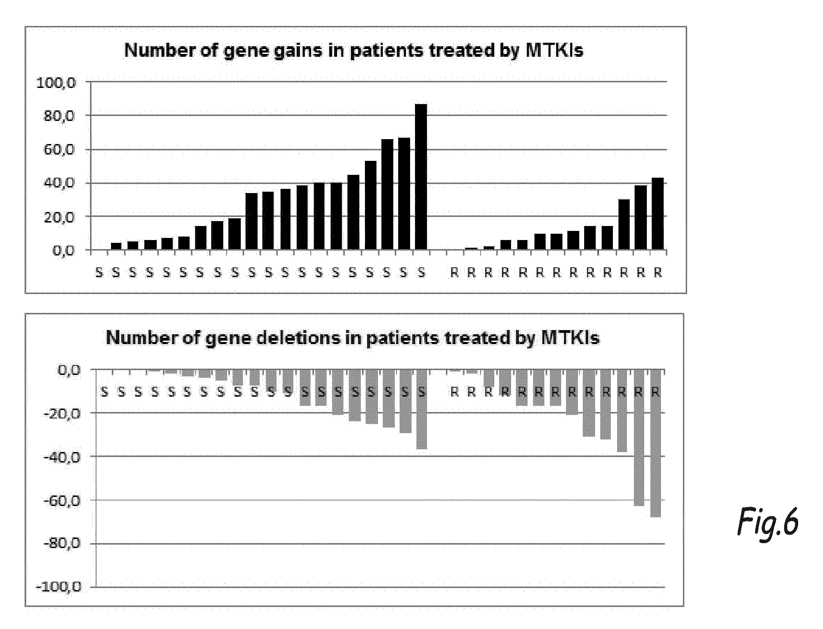

We then compared whole-genome gains and losses of sensitive tumor profiles (n=20) and resistant tumor profiles (n=13), and found that a total of 622 gains in sensitive tumors (mean: 31.1; range0-87) versus 186 gains in resistant tumors (mean:14.3; range 0-43) (p =0.053, Mann-Whitney), and 247 losses in 20 sensitive samples (mean: 12.4; range0 -37) versus 327 losses in resistant samples(mean: 25.2; range 1-68)(P =0.036; Mann-Whitney). The difference of TTC and TTL was marginally different between sensitive and resistant tumors (P=0.06; Mann-Whitney). **Fig. 6****.**

### Second line and beyond

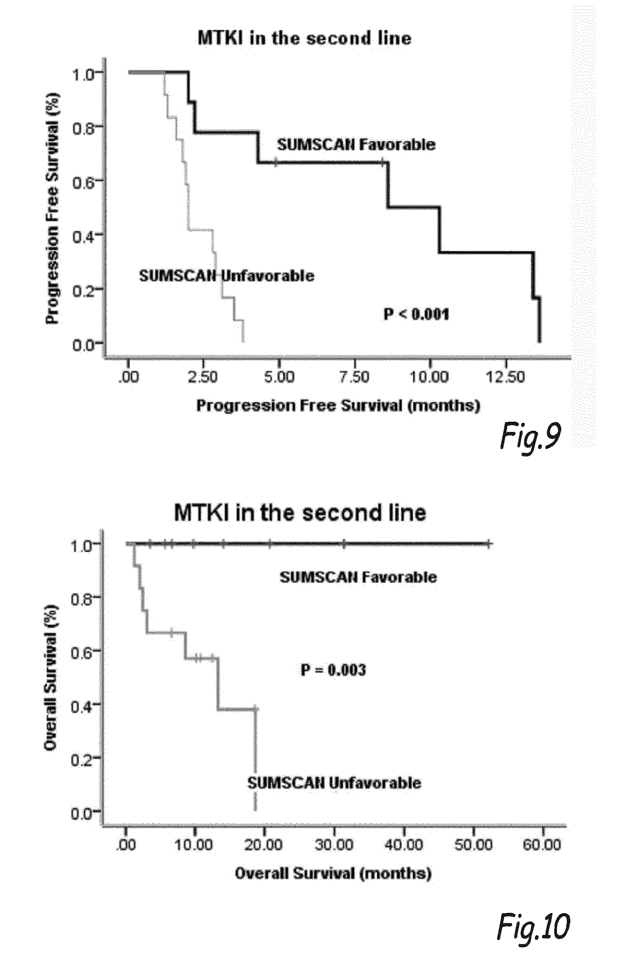

The relevance of SUMSCAN was analyzed in 22 of the 33 patients treated with 2^{nd} line or more MTKIs. Three patients were treated with more than 2 MKTI. We had included also the 3^{rd} line and 4^{th} line MTKI in the analysis. This third validation cohort consists of 26 cases treated by a second line MTKI or beyond. SUMSCAN predicted 10 out of 12 MTKIs sensitive patients in second line and 12 out of 14 MTKIs resistant patients in second line, with an accuracy rate of 84.6% (22/26), a sensitivity of 83.3% and a specificity of 85.7% (P = 0.0011, Fisher exact test). Interestingly, two patients had a TTC superior to that of the 1^{st} line MKTI, both had experienced a longer PFS than that in the 1^{st} line setting, one with thyroid carcinoma had a PFS of 19 months with vandetanib (TTC =3) and 35 months with sunitinib (TTC = 6) in the 2^{nd} line. Patients with a favorable SUMSCAN profile had a significantly better PFS and OS (**Fig. 9 and 10**)

### SUMSCAN performance in 5 histological types treated by MTKIs

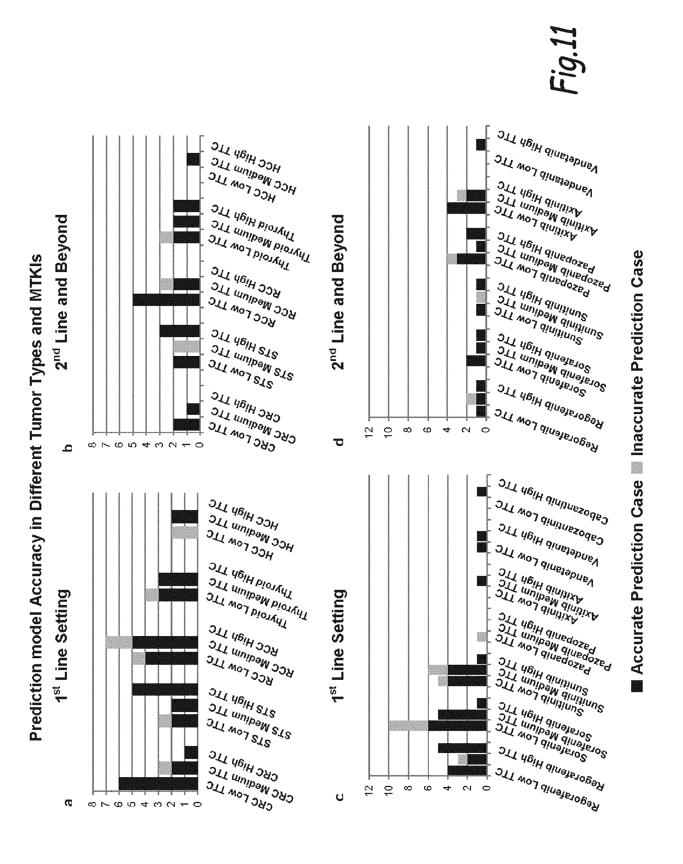

See **Fig. 11****.** Each histological subgroup is divided into 3 groups according to TTC (TTC≤1; TTC =2, 3; TTC≥4) from left to right. The black bar shows the total number of patients for whom SUMSCAN succeed to predict the clinical outcome. The grey bar shows the number of patients for whom SUMSCAN failed to predict the clinical outcome. Figure 11b, SUMSCAN performance in 5 histological types treated by MTKI in the 2nd line. Figure 11c, SUMSCAN performance in 7 MTKIs applied as 1 st line MTKI treatment. Figure 11d, SUMSCAN performance in 7 MTKIs applied as 2nd line MTKI treatment. As shown in figure 11, no discordant cases observed in high TTC tumors across different histological types and different MTKI in all line setting.

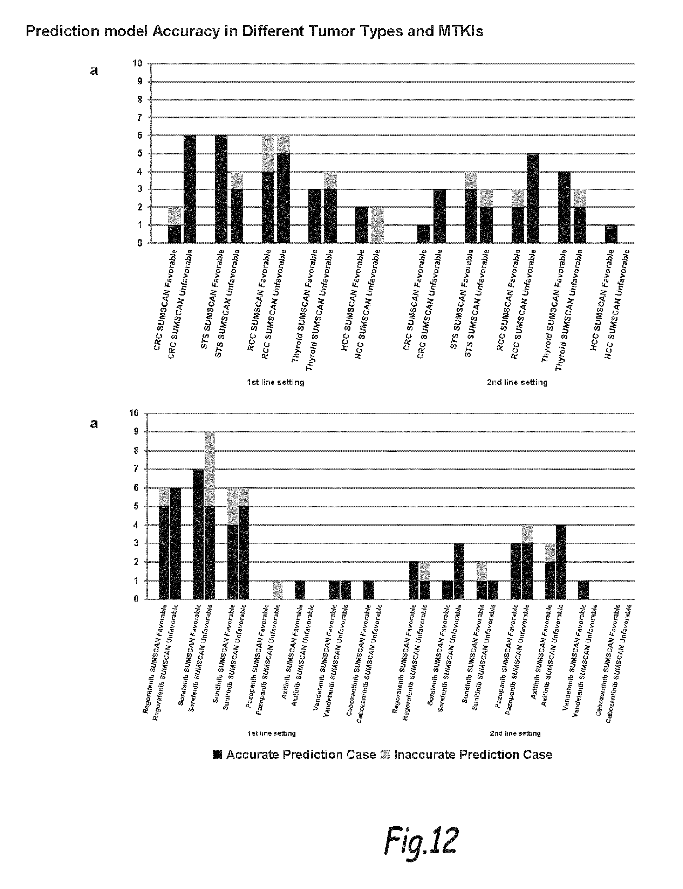

See **Fig. 12****.** SUMSCAN performance in 5 histological types treated by MTKI in the 1st line and 2nd line and beyond. Each histological subgroup is divided into 2 groups according to SUMSCAN (Predicted sensitive if presenting a favorable SUMSCAN profile; predicted Unfavorable if presenting an unfavorable SUMSCAN profile) from left to right CRC, STS, RCC, Thyroid carcinoma and HCC. The black bar shows the number of patients for whom SUMSCAN succeed to predict the clinical outcome. The grey bar shows the total number of patients for whom SUMSCAN failed to predict the clinical outcome. Figure 12b, SUMSCAN performance across 7 MTKI used in the 1st line and 2nd line. From left to right: regorafenib, sorafenib, sunitinib, pazopanib, axitinib, vandetanib and cabozantinib.

### SUMSCAN is not predictive of the response to conventional chemotherapy

To evaluate the specificity of the SUMSCAN algorithm, the correlation between the SUMSCAN and response to conventional chemotherapy regimens (irinotecan and oxaliplatin containing regime) received beforehand was evaluated in 21 CRC patients. These patients were divided into 2 groups according to the SUMSCAN (predicted sensitive, predicted resistant; see **Table 10**). No correlation between the SUMSCAN and response to conventional chemotherapy were observed.

**Table 10: Prediction model and response to chemotherapy in CRC patients (n = 21)**

| Irinotecan Sensitivity | Predicted Sensitive | Predicted Resistant | Total |
|---|---|---|---|
| Sensitivity | 9 (42.9%) | 7 (33.3%) | 16 (76.2%) |
| Resistant | 3 (14.3%) | 2 (9.5%) | 5 (23.8%) |
| P = 0.882 (Chi-square) | | | |

| Oxaliplatin Sensitivity | Predicted Sensitive | Predicted Resistant | Total |
|---|---|---|---|
| Sensitivity | 6 (28.6%) | 7 (33.3%) | 13 (61.9%) |
| Resistant | 6 (28.6%) | 2 (9.5%) | 8 (38.1%) |
| P = 0.195 (Chi-square) | | | |

### Discussion

The hypothesis explored in this work was that the antitumor activity of MTKI may be related to the sum of gains and losses of genes encoding for the receptors and targets of these MTKI in a given tumor. The underlying biological rationale is that a high level of target genes copy number gains in tumor may indicate tumor's oncogenes dependence and therefore their increased sensitivity to molecules targeting these genes.

Actually, standard biomarkers of response to MTKI, in particular those inhibiting VEGF receptors, have not been identified yet. This is in contrast with what is observed when a key driver event such as KIT mutations, BRAF mutations, ALK translocations is present in the tumor.

Here, it is reported that the antitumor activity of MTKIs in tumors lacking a well-defined strong oncogenic driver is strongly correlated to the gains of additional copies and/or losses of genes encoding for the protein kinases which are the targets of these MTKIs. The sum of gains of the genes encoding for targets of MKTIs, termed as tumor target charge (TTC) was found higher in responding patients, enabling to delineate a predictive score. Based on the concept of TTC, we created the predictive score SUMSCAN, identifying a favorable and an unfavorable group based on the classification of the patients into three TTC groups (low, medium and high).

Regorafenib was investigated firstly in patients with mCRC progressing after irinotecan, oxaliplatin and 5FU. The difference between TTC and TTL was found significantly higher in patients who experienced tumor control to regorafenib. This was confirmed in the first validation series including mCRC patients and advanced STS progressing after standard chemotherapy with doxorubicin. A predictive score SUMSCAN was delineated and validated on both series. The PFS and OS of these 2 series were significantly longer in patients with a favorable SUMSCAN. Importantly, SUMSCAN predicted exclusively efficacy to MTKI: no correlation was observed with the previous response to widely used irinotecan and/or oxaliplatin containing regimens in mCRC treatment.

The generalizability of SUMSCAN in other tumor types and other MTKI has been tested. All 33 patients included in ProfilER and pretreated with MTKI were analyzed using the same strategy. These included a variety of histological subtypes and a six different MTKIs (sorafenib, sunitinib, pazopanib, axitinib, vandetanib and cabozantinib). The presence of gains and deletions of target genes of each of these six MTKIs was determined for each patient and TTC as well as the SUMSCAN score could be identified for each patient and each line of treatment. Again, the sum of gains of genes encoding for targets of the MTKIs were higher in responding patients across this variety of histological types and MTKIs. The SUMSCAN score was predictive of response and tumor control, and was a powerful prognostic factor for PFS and OS in this series.

This was further confirmed when the response to the second line MTKI treatment was assessed in the subgroup of 22 of these 33 patients. In these patients, response and tumor control to the second line MTKI was significantly better when a favorable SUMSCAN score was observed, with a short PFS (median= 2.8 months) in patients with an unfavorable SUMSCAN score across histological subtypes and MTKI.

Finally, in all the series, all patients with more than 4 gains in genes encoding for targets of the MTKI derived clinical benefit.

Genomic alterations of several of the individual genes encoding for MTKI targets were found predictive of clinical benefit, but none were as discriminant as the numerical combination of TTC and TTL described in the SUMSCAN model. In an attempt to understand the false positive of the SUMSCAN score in the series of patients treated regorafenib, the presence of specific mutations within the NGS panel of the ProfiLER trial was investigated: only PIK3CA mutations were found exclusively observed in non-responders to regorafenib, although 2 of these patients had a favorable SUMSCAN score. No other mutations were associated with response, including KRAS, Tp53. These findings suggest that PIK3CA mutation could further refine the SUMSCAN prediction for regorafenib resistance. It was also opbserved that the mutation frequencies inversely correlate with the copy number alterations in mCRC. This trend is referred as cancer genome hyperbola which is initially to describe the fact that tumors at the extremes of genomic instability had either a large number of somatic mutations or a large number of copy number alterations, never both.

An important observation is that the overall number of gene gains in the whole genome was higher in responding patients, while conversely the number of gene losses tended to be higher in progressive patients. Even though the difference was less significant when target genes of individual MTKI were excluded from the comparison, this observation suggest that tumors which respond to these MTKIs are characterized by a global "gain profile", with more proto-oncogene copies, including those coding for target proteins of these MTKIs. This was not observed for oxaliplatin or irinotecan response in mCRC (not shown). These tumors with a "gain profile" may therefore be better candidate for therapies targeting multiple oncogenes in general.

These results also challenge the antiangiogenic role of these MTKIs as a major component of their antitumor activity. Indeed, while prolonged clinical benefit was observed in patients whose tumor do not present gains of the target genes, the analysis of the SUMSCAN score and TTC of individual tumor suggests that the antitumor activity of these agents is exerted primarily on the tumor cells which gained additional copies of genes encoding for MTKI targets during the process of acquisition of genomic alteration. This question is also of importance for patients treated with regorafenib or sunitinib for a GIST, with well-identified key molecular. This question is currently explored in a large dataset of patients treated in 2nd or more line with these MTKI is currently explored. It is important to note that the antitumor activity of regorafenib was observed at the same level regardless of the nature of the KIT /or PDGFRA mutation¹⁸. In conclusion, these results point to a novel concept that the response to any MTKI in human solid tumors is influenced by the sum of gains and losses of the genes encoding for the protein targets of these MTKI in the tumor. A predictive model for the selection of patients is presented and proposed for future evaluation in other series. These results could have important consequences for a better selection of patient candidate for these treatments in routine clinical settings. In addition, these results and the GSH method disclosed herein may be useful to identify candidate patients for these MTKIs outside the approved indications enabling registration of an already approved agent in additional indications, or enabling a non-approved agent to be registered. Finally, this concept that the sum of gains and losses of genes coding for target proteins is predictive for treatment efficacy has broader application beyond MTKI targeting VEGFR: the identification of responders and refractory patient to multitargeted inhibitors of ALK MET, SRC, mTOR/PI3KCA/AKT, Src family of kinases for instance is made using this method.

### REFERENCES

1. Demetri, G. D., et al. Proc Am Soc Clin Oncol, 2005, Vol. 23, p. 4000a.
2. Motzer, R. J. et al. New England Journal of Medicine, 2007, 356(2), 115-124.
3. Llovet, J. M. et al. New England Journal of Medicine, 2008, 359(4), 378-390.
4. Cheng, A. L. et al. The lancet oncology, 2009, 10(1), 25-34.
5. Grothey, A. et al. The Lancet, 2013, 381(9863), 303-312.
6. Carr, L. L. et al. Clinical Cancer Research, 2010, 16(21), 5260-5268.
7. Sternberg, C. N. et al. Journal of Clinical Oncology, 2010, 28(6), 1061-1068.
8. Cohen, E. E. et al. Journal of Clinical Oncology, 2008, 26(29), 4708-4713.
9. Demetri, G. D. et al. The Lancet, 2006, 368(9544), 1329-1338.
10. Hanahan, D., & Weinberg, R. A. et al. Cell, 2011, 144(5), 646-674.
11. Davoli, T. et al. Cell, 2013, 155(4), 948-962.
12. Druker, B. J. et al. New England Journal of Medicine, 2001, 344(14), 1031-1037.
13. Heinrich, M. C. et al. Journal of Clinical Oncology, 2007, 21(23), 4342-4349.
14. Debiec-Rychter, M. et al. European Journal of Cancer, 2004, 40 (5), 689-695.
15. Miller, A. J., & Mihm Jr, M. C. New England Journal of Medicine, 2006, 355(1), 51-65.
16. Flaherty, K. Tet al. New England Journal of Medicine, 2010, 363(9), 809-819.
17. Kelleher, F. C., & McDermott, R. European Journal of Cancer, 2010, 46(13), 2357-2368.
18. Demetri, G. D. et al. The Lancet, 2013, 381(9863), 295-302.
19. George, S., et al. J Clin Oncol, 29(15 May 2011 Supplement), 10007.
20. Peña, C. et al. Clinical cancer research, 2010, 16(19), 4853-4863.
21. Zhang, Z et al. BMC medicine, 2009, 7(1), 41.
22. Porta, C. et al. Kidney international, 2010, 77(9), 809-815.
23. Gruenwald, V. et al. BMC cancer, 2010, 10(1), 695.
24. Xu, C. F et al. Journal of clinical oncology, 2011, JCO-2010.
25. Tran, H. T. et al. The lancet oncology, 2012, 13(8), 827-837.
26. Lenz, H. J. et al. J Clin Oncol, 2013, 31, abstract-3514.
27. Meyerson, M., Gabriel, S. & Getz, G. (2010. Nature Reviews Genetics, 2010, 11(10), 685-696.
28. Stratton, M. R., Campbell, P. J., & Futreal, P. A. Nature, 2009, 458(7239), 719-724.
29. Vogelstein, B. et al. Science, 2013, 339(6127), 1546-1558.
30. Eisenhauer, E. A., Therasse, P., Bogaerts, J., Schwartz, L. H., Sargent, D., Ford, R., & Verweij, J. (2009). New response evaluation criteria in solid tumours: revised RECIST guideline.
31. http://www.drugbank.ca/
32. Ciriello, G. et al. Nature genetics, 2013, 45(10), 1127-1133.

## Claims

1. A method for predicting the anti-tumor response in a human or animal having a tumor to multiple kinase inhibitors, using any multiple kinase inhibitor, comprising:
- selection of genes encoding for protein kinases targeted by the said tyrosine kinase inhibitor,
- for each one of these genes, providing at least one nucleic acid probe which hybridizes to said gene under stringent conditions, thus providing an array of nucleic acid probes,
- having a biological sample containing cancer cells from said human or animal,
- extracting DNA from the sample, fragmenting into DNA fragments, optionally labeling the DNA fragments,
- submitting the optionally labeled DNA fragments to hybridization with the array of nucleic acid probes,
- recovering and quantifying for all the genes the gains or losses in gene copy numbers,
- wherein gains and losses of gene copy numbers of each selected gene are used to determine whether the tumor is sensitive or not to said kinase inhibitor.

2. The method of claim 1, wherein the array of nucleic acid probes is an array of oligonucleotides.

3. The method of claim 1 or 2, comprising mixing the optionally labeled DNA fragments with optionally labelled non-tumoral reference DNA and subjecting the mixture to Comparative Genomic Hybridization (CGH).

4. The method of claim 3, wherein CGH is performed using a human whole-genome oligonucleotide array.

5. The method of any one of claims 1 to 4, wherein:
- the sum TTC of the copy-number gains of all selected genes encoding for protein kinases targeted by said kinase inhibitor is calculated,
- the sum TTL of the copy-number losses of all selected genes encoding for protein kinases targeted by said kinase inhibitor is calculated,
- wherein a sum of the copy-number gains greater than the sum of copy-number losses is indicative that the tumor is sensitive to the kinase inhibitor.

6. The method of claim 5, wherein:
- a TTC ≥ 4 and TTC ≥ TTL is indicative that the tumor is sensitive to the kinase inhibitor;
- a TTC ≥ 4 and TTC < TTL is indicative that the tumor is resistant to the kinase inhibitor;
- a TTC such as 1 <TTC<4 combined to TTC > TTL is indicative that the tumor is sensitive to the kinase inhibitor
- a TTC such as 1 <TTC<4 combined to TTC ≤ TTL is indicative that the tumor is resistant to the kinase inhibitor; and/or
- a TTC ≤ 1 is indicative that the tumor is resistant to the kinase inhibitor.

7. The method of claim 5, wherein the following algorithm is used:

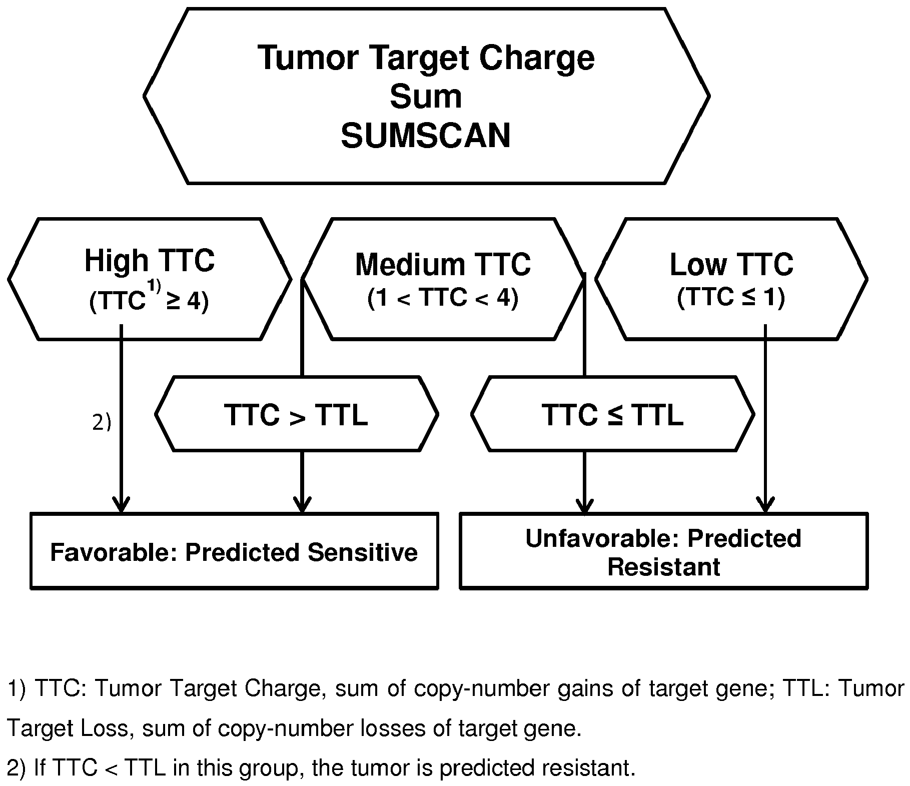

8. The method of any one of the preceding claims, wherein CGH is an array comparative genomic hybridization.

9. The method of any one of the preceding claims, wherein the kinase inhibitor is regorafenib, imatinib, sorafenib, sunitinib, pazopanib, axitinib, cabozantinib or vandetanib.

10. The method of claim 9, wherein the kinase inhibitor is regorafenib and the genes are selected from the group consisting of ieRET, (VEGFR1(FLT1), VEGFR2(KDR), VEGFR3(FLT4), KIT, PDGFRα, PDGFRβ, FGFR1, FGFR2, angiopoietin-1 receptor(TEK), DDR2, High affinity nerve growth factor receptor(NTRK1), EPHA2, RAF1, BRAF, MAPK11, FRK and ABL1.

11. A multi-kinase inhibitor for use in treating a cancer in a human or animal that has been predicted as sensitive to the multi-kinase inhibitor.

12. The inhibitor for the use of claim 11, wherein the human or animal that has been predicted as sensitive to the multi-kinase inhibitor by Comparative Genomic Hybridization (CGH).

13. The inhibitor for the use of claim 12, wherein the human or animal that has been predicted as sensitive to the multi-kinase inhibitor by the method according to any one of claims 1 to 9.

14. The inhibitor for the use of claim 12, wherein the human or animal that has been predicted as sensitive to the multi-kinase inhibitor by Comparative Genomic Hybridization (CGH) and use of the following algorithm :
